# EUROPEAN PATENT APPLICATION

(11) **EP 3 450 963 A1**
(43) Date of publication of application: **06.03.2019**
(21) Application number: 17789006.8
(22) Date of filing: 10.02.2017
(51) Int. Cl.: G01N 21/64, G01N 15/14, G01N 21/78, G01N 33/48, G01N 33/53, C12Q 1/02

(54) **FLUORESCENT PROBE, FLUORESCENCE DETECTION METHOD, AND METHOD FOR USING FLUORESCENT PROBE**

(30) Priority: 28.04.2016 JP 2016091359
(71) Applicant: National University Corporation Nagoya University, Nagoya-shi, Aichi 464-8601 (JP); Kurashiki Boseki Kabushiki Kaisha, Kurashiki-shi, Okayama 710-0054 (JP)
(72) Inventor: HAYASHI, Koichiro, Nagoya-shi, Aichi 464-8601 (JP); SAKAMOTO, Wataru, Nagoya-shi, Aichi 464-8601 (JP); YOGO, Toshinobu, Nagoya-shi, Aichi 464-8601 (JP); MARUOKA, Hiroki, Neyagawa-shi, Osaka 572-0823 (JP)
(74) Representative: Dehns
(86) International application number: PCT/JP2017/004981
(87) International publication number: WO 2017/187717

(57) **Abstract**

The present invention relates to a fluorescent probe including a carrier molecule, a fluorescent dye a bound to the carrier molecule, and a fluorescent dye b bound to the carrier molecule in which the excitation wavelengths of the fluorescent dyes a and b are different, and FRET does not occur between the fluorescent dyes a and b. The present invention also relates to a method for detecting fluorescence that includes a step of labeling target cells with the fluorescent probe, and a step of irradiating the target cells labeled with the fluorescent probe with excitation light and observing the fluorescence from the fluorescent probe. The present invention also relates to a method for using a fluorescent probe that includes a step of fluorescently labeling cells with the fluorescent probe, a step of screening the fluorescence-labeled cells using a flow cytometer or a fluorescence microscope, a step of transplanting the screened fluorescence-labeled cells into a living organism, and a step of observing the fluorescence-labeled cells in the living organism using an in vivo fluorescence imaging apparatus.

## Description

### Technical Field

The present invention relates to a fluorescent probe including two types of fluorescent dyes, a method for detecting fluorescence, and a method for using a fluorescent probe.

### Background Art

In recent years, in vivo fluorescence imaging analysis using animals such as mice has been widely performed in the field of cancer research, embryological research, and the like. The fluorescence imaging analysis is a major technique for analyzing information about positions at which cells such as stem cells including iPS cells, ES cells, and the like and disease-related cells including cancer cells, cirrhotic cells, and the like differentiate, grow, and metastasize after being transplanted into mice. Under current circumstances, only methods using the fluorescence imaging analysis can be used to observe the progression of transplanted cells, particularly with animals such as mice that are still alive, and such methods are regarded as particularly important analysis methods.

In general, fluorescence imaging analysis on transplanted cells in vivo is performed as follows: cells are labeled with a fluorescent probe in vitro in advance before the cells are transplanted, the cells are transplanted into a living organism such as a mouse, and the cells are observed using an in vivo fluorescence imaging apparatus. For example, Patent Document 1 discloses use of a porphyrin-containing complex obtained by binding anionic porphyrin, cationic organoalkoxysilane, and non-cationic silane together as a near-infrared fluorescent probe for observation of a living organism.

On the other hand, when a predetermined number of cells are labeled with a fluorescent probe, it is common that not all of the cells are labeled with the fluorescent probe. Therefore, in some cases, cells that are not bound to the fluorescent probe are also transplanted into a living organism such as a mouse. Under current circumstances, the fluorescence of the near-infrared fluorescent probe for observation of a living organism cannot be detected using an in vitro fluorescence imaging apparatus such as a fluorescence microscope or a flow cytometer, and it is difficult to observe fluorescence-labeled cells bound to the near-infrared fluorescent probe in vitro. The reason for this is that an in vitro fluorescence imaging apparatus and an in vivo fluorescence imaging apparatus require different wavelength characteristics. Analysis using an in vitro fluorescence imaging apparatus is generally performed using an excitation wavelength region of 350 to 650 nm and a fluorescence wavelength region of 400 to 800 nm, and analysis using an in vivo fluorescence imaging apparatus is generally performed using an excitation wavelength region of 600 to 850 nm and a fluorescence wavelength region of 650 to 920 nm.

### Citation List

### Patent Document

Patent Document 1: JP 2013-136555A

### Disclosure of Invention

### Problem to be Solved by the Invention

As described above, the wavelength characteristics of a fluorescent dye suitable for in vitro fluorescence imaging analysis are significantly different from the wavelength characteristics of a fluorescent dye suitable for in vivo fluorescence imaging analysis. Therefore, under current circumstances, there are no fluorescent probes that can be used in both in vitro fluorescence imaging analysis and in vivo fluorescence imaging analysis.

In order to solve the foregoing conventional problems, the present invention provides a fluorescent probe that can be used in both in vitro fluorescence imaging analysis and in vivo fluorescence imaging analysis, a method for detecting fluorescence, and a method for using a fluorescent probe.

### Means for Solving Problem

The present invention relates to a fluorescent probe including a carrier molecule, a fluorescent dye a bound to the carrier molecule, and a fluorescent dye b bound to the carrier molecule, wherein excitation wavelengths of the fluorescent dyes a and b are different, and fluorescence resonance energy transfer (FRET) does not occur between the fluorescent dyes a and b.

It is preferable that the fluorescent probe is a cell labeling fluorescent probe, the fluorescent dye a is a fluorescent dye for in vitro fluorescence imaging, and the fluorescent dye b is a fluorescent dye for in vivo fluorescence imaging. A configuration may be employed in which the fluorescent probe is specifically bound to a surface of a cell so that the cell is labeled, or a configuration may be employed in which the fluorescent probe is taken up by a cell so that the cell is labeled.

It is preferable that an excitation wavelength of the fluorescent dye a is in a range of 350 to 650 nm, and an excitation wavelength of the fluorescent dye b is in a range of 600 to 850 nm. It is preferable that the fluorescent dye a is porphyrin. It is preferable that the fluorescent dye b is indocyanine green.

It is preferable that the carrier molecule is polysiloxane.

The fluorescent probe may be used in screening of fluorescence-labeled cells using a flow cytometer.

The present invention also relates to a method for detecting fluorescence used to observe target cells, and the method includes a step of labeling the target cells with a fluorescent probe, and a step of irradiating the target cells labeled with the fluorescent probe with excitation light and observing fluorescence from the fluorescent probe, wherein the fluorescent probe includes a carrier molecule, a fluorescent dye a bound to the carrier molecule, and a fluorescent dye b bound to the carrier molecule, the excitation wavelengths of the fluorescent dyes a and b are different, and fluorescence resonance energy transfer does not occur between the fluorescent dyes a and b.

A configuration may be employed in which the target cells labeled with the fluorescent probe are irradiated with excitation light that excites the fluorescent dye a, and fluorescence from the fluorescent probe is observed through in vitro fluorescence imaging. A configuration may be employed in which the target cells labeled with the fluorescent probe are transplanted into a living organism (excluding a human), the living organism is irradiated with excitation light that excites the fluorescent dye b, and fluorescence from the fluorescent probe is observed through in vivo fluorescence imaging.

The present invention also relates to a method for using the above-mentioned fluorescent probe, and the method includes a step of fluorescently labeling cells with the fluorescent probe, a step of confirming and screening the fluorescence-labeled cells that are fluorescently labeled with the fluorescent probe using a flow cytometer or a fluorescence microscope, a step of transplanting the screened fluorescence-labeled cells into a living organism (excluding a human), and a step of observing the fluorescence-labeled cells in the living organism using an in vivo fluorescence imaging apparatus, wherein the screening of the fluorescence-labeled cells using a flow cytometer is performed by detecting fluorescence originating from the fluorescent dye a, and the observation of the fluorescence-labeled cells in the living organism using an in vivo fluorescence imaging apparatus is performed by detecting fluorescence originating from the fluorescent dye b.

### Effects of the Invention

With the present invention, using the fluorescent probe, including the fluorescent dyes a and b whose excitation wavelengths are different and between which fluorescence resonance energy transfer does not occur, makes it possible to perform both in vitro fluorescence imaging analysis and in vivo fluorescence imaging analysis. In particular, in vitro fluorescence imaging analysis can be performed on cells using existing in vitro fluorescence imaging apparatuses such as fluorescence microscopes and flow cytometers, and in vivo fluorescence imaging analysis can be performed in vivo using existing in vivo fluorescence imaging apparatuses. It is also possible to screen fluorescence-labeled cells labeled with the fluorescent probe using a flow cytometer, transplant the screened fluorescence-labeled cells into a living organism such as a mouse, and observe the localization of the fluorescence-labeled cells in the living organism such as a mouse over time.

### Brief Description of Drawings

[FIG. 1] FIG. 1 shows FT-IR spectra of tetrakis(4-carboxyphenyl)porphyrin (TCPP) and a hybrid nanoparticle (PPC HNPs) produced through hydrolysis and condensation of TCPP and (3-mercaptopropyl)trimethoxysilane (MPTMS) measured using a Fourier transform infrared spectrophotometer (FT-IR).
[FIG. 2] FIG. 2 shows a ²⁹Si solid-state nuclear magnetic resonance (solid-state NMR) spectrum of PPS HNPs.
[FIG. 3] FIG. 3 shows a TG curve and a DTA curve of PPS HNPs.
[FIG. 4] FIG. 4A shows an absorption spectrum of a supernatant after FA-PEG/ICG-PPS HNPs production reaction, and FIG. 4B shows a calibration curve for a folic acid concentration.
[FIG. 5] FIG. 5 shows absorption spectra of PPS HNPs and a complex (FA-PEG/ICG-PPS HNPs) obtained by further binding indocyanine green (ICG) and folic acid (FA) to PPS HNPs.
[FIG. 6] FIG. 6A shows an excitation spectrum and a fluorescence spectrum of FA-PEG/ICG-PPS HNPs on a short wavelength side, and FIG. 6B shows an excitation spectrum and a fluorescence spectrum of FA-PEG/ICG-PPS HNPs on a long wavelength side.

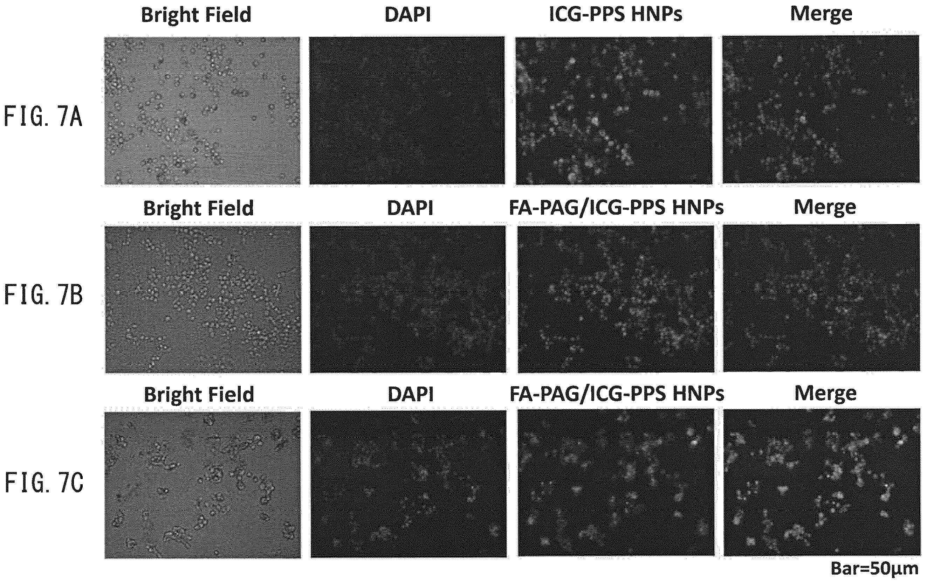

[FIG. 7] FIG. 7A shows a bright field image (Bright field) of RAW264.7 cells derived from a mouse macrophage, a fluorescence image (DAPI) of RAW264.7 cells stained with DAPI captured using a fluorescence microscope, a fluorescence image (ICG-PPS HNPs) of RAW264.7 cells labeled with a complex (ICG-PPS HNPs) obtained by further binding indocyanine green (ICG) to PPS HNPs captured using a fluorescence microscope, and an image (Merge) obtained by merging these three images together. FIG. 7B shows a bright field image (Bright field) of HeLa S3 cells derived from human cervical cancer, a fluorescence image (DAPI) of HeLa S3 cells stained with DAPI captured using a fluorescence microscope, a fluorescence image (FA-PEG/ICG-PPS HNPs) of HeLa S3 cells labeled with FA-PEG/ICG-PPS HNPs captured using a fluorescence microscope, and an image (Merge) obtained by merging these three images together. FIG. 7C shows a bright field image (Bright field) of HCT116 cells derived from human large intestine cancer, a fluorescence image (DAPI) of HCT116 cells stained with DAPI captured using a fluorescence microscope, a fluorescence image (FA-PEG/ICG-PPS HNPs) of HCT116 cells labeled with FA-PEG/ICG-PPS HNPs captured using a fluorescence microscope, and an image (Merge) obtained by merging these three images together.
[FIG. 8] FIG. 8 shows a result of analysis of RAW264.7 cells labeled with ICG-PPS HNPs using a flow cytometer.
[FIG.9] FIG. 9 shows a result of analysis of HeLa S3 cells labeled with FA-PEG/ICG-PPS HNPs using a flow cytometer.
[FIG. 10] FIG. 10 shows a result of analysis of HCT116 cells labeled with FA-PEG/ICG-PPS HNPs using a flow cytometer.
[FIG. 11] FIG. 11 shows a result of observation of the fluorescence-labeled cell localization over time in a mouse body into which RAW264.7 cells labeled with ICG-PPS HNPs were transplanted using an in vivo fluorescence imaging apparatus.
[FIG. 12] FIG. 12 shows a result of observation of the fluorescence-labeled cell localization over time in a mouse body into which HeLa S3 cells labeled with FA-PEG/ICG-PPS HNPs were transplanted using an in vivo fluorescence imaging apparatus.
[FIG. 13] FIG. 13 shows fluorescence images of organs of a mouse observed using an in vivo fluorescence imaging apparatus 24 hours after RAW264.7 cells labeled with ICG-PPS HNPs were transplanted.
[FIG. 14] FIG. 14 shows results from organs of a mouse analyzed using an in vivo fluorescence imaging apparatus 24 hours after RAW264.7 cells labeled with ICG-PPS HNPs were transplanted.
[FIG. 15] FIG. 15 shows fluorescence images of organs of a mouse observed using an in vivo fluorescence imaging apparatus 24 hours after HeLa S3 cells labeled with FA-PEG/ICG-PPS HNPs were transplanted.
[FIG. 16] FIG. 16 shows results from organs of a mouse analyzed using an in vivo fluorescence imaging apparatus 24 hours after HeLa S3 cells labeled with FA-PEG/ICG-PPS HNPs were transplanted.

### Description of the Invention

As a result of intensive research on a fluorescent probe that can be used in both in vitro fluorescence imaging analysis and in vivo fluorescence imaging analysis, the inventors of the present invention found that, in a fluorescent probe including a carrier molecule, and a fluorescent dye a bound to the carrier molecule and a fluorescent dye b bound to the carrier molecule, fluorescent dyes whose excitation wavelengths are different and between which fluorescence resonance energy transfer does not occur could be used as the fluorescent dyes a and b to obtain a fluorescent probe that can be used in both in vitro fluorescence imaging analysis and in vivo fluorescence imaging analysis, and the present invention was thus achieved. Conventionally, there are fluorescent probes including two types of fluorescent dyes, but these fluorescent probes cannot be used in both in vitro fluorescence imaging analysis and in vivo fluorescence imaging analysis because fluorescence resonance energy transfer occurs between the two types of fluorescent dyes.

### Fluorescent probe

A fluorescent probe of the present invention includes a carrier molecule, a fluorescent dye a bound to the carrier molecule, and a fluorescent dye b bound to the carrier molecule. The excitation wavelengths of the fluorescent dyes a and b are different, and fluorescence resonance energy transfer does not occur between the fluorescent dyes a and b. It is preferable that the fluorescent dyes a and b are covalently bound to the carrier molecule.

It is preferable that the fluorescent dye a is a fluorescent dye for in vitro fluorescence imaging (also referred to as "fluorescent dye for visible light fluorescence imaging"). It is more preferable that the excitation wavelength is in a range of 350 to 650 nm, and the fluorescence wavelength is in a range of 400 to 800 nm, and it is even more preferable that the excitation wavelength is in a range of 380 to 580 nm, and the fluorescence wavelength is in a range of 450 to 680 nm. When the excitation wavelength and the fluorescence wavelength are in the above-described ranges, fluorescence-labeled cells labeled with the fluorescent probe can be observed using a general-purpose in vitro fluorescence imaging apparatus such as a fluorescence microscope or a flow cytometer.

Fluorescent dyes for in vitro fluorescence imaging such as porphyrin, fluorescein, and rhodamine can be used as the fluorescent dye a, for example. There is no particular limitation on the type of porphyrin, but porphyrin having a carboxyl group can be used from the viewpoint of being suitable as a fluorescent dye for in vitro fluorescence imaging. It should be noted that the term "porphyrin" is used to collectively refer to a ring compound in which four pyrrole rings are alternately bound to four methine groups at α positions, and derivatives thereof. The excitation wavelength of porphyrin having a carboxyl group is generally in a range of 400 to 650 nm, and its fluorescence wavelength is in a range of 600 to 740 nm.

For example, a compound represented by General Formula (I) below can be used as the porphyrin having a carboxyl group.

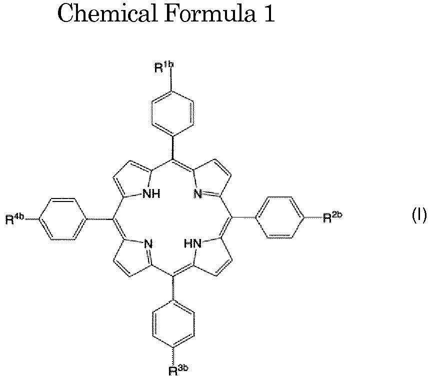

In General Formula (I) above, R^{1b}, R^{2b}, R^{3b}, and R^{4b} are optionally the same or different and represent a carboxyl group (COOH), a sulfo group (SO₃H), or a hydrogen atom (H) (it should be noted that a case where all of these are hydrogen atoms and a case where all of these are sulfo groups are excluded). It is preferable that, in General Formula (I) above, all of R^{1b}, R^{2b}, R^{3b}, and R^{4b} are carboxyl groups, or R^{1b} is a carboxyl group and R^{2b}, R^{3b}, and R^{4b} are hydrogen atoms. It is more preferable to use, as the porphyrin having a carboxyl group, a compound that is represented by General Formula (I) above and in which all of R^{1b}, R^{2b}, R^{3b}, and R^{4b} are carboxyl groups. The compound in which all of R^{1b}, R^{2b}, R^{3b}, and R^{4b} are carboxyl groups is called tetrakis(4-carboxyphenyl)porphyrin (TCPP).

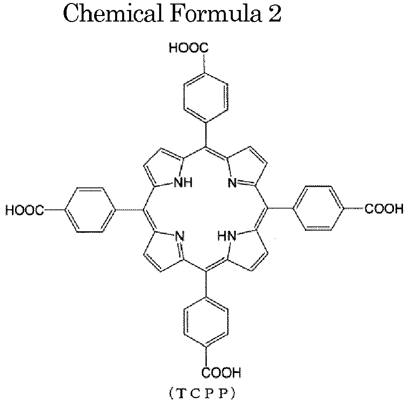

In addition, bilirubin, hemin, protoporphyrin, and the like can also be used as the porphyrin having a carboxyl group, for example.

The porphyrin having a carboxyl group used in the present invention is a known compound or can be easily manufactured using a known method. For example, commercially available compounds can be obtained from Tokyo Chemical Industry Co., Ltd., and the like.

A fluorescent dye whose excitation wavelength is different from that of the fluorescent dye a is used as the fluorescent dye b, and fluorescence resonance energy transfer does not occur between the fluorescent dyes a and b. It is preferable that the fluorescent dye b is a fluorescent dye for in vivo fluorescence imaging (also referred to as "fluorescent dye for near-infrared fluorescence imaging"). It is more preferable that the excitation wavelength is in a range of 600 to 850 nm, and the fluorescence wavelength is in a range of 650 to 920 nm, and it is even more preferable that the excitation wavelength is in a range of 630 to 790 nm, and the fluorescence wavelength is in a range of 680 to 910 nm. When the excitation wavelength and the fluorescence wavelength are in the above-described ranges, fluorescence-labeled cells labeled with the fluorescent probe in a living organism such as a mouse can be observed using a general-purpose in vivo fluorescence imaging apparatus.

Fluorescent dyes for in vivo fluorescence imaging such as indocyanine compounds, coumarin, rhodamine, xanthene, hematoporphyrin, and fluorescamine can be used as the fluorescent dye b, for example. It is preferable to use indocyanine green or a derivative thereof from the viewpoint of safety in a living organism. In general, the excitation wavelength of indocyanine green is in a range of 740 to 790 nm, and its fluorescence wavelength is in a range of 810 to 860 nm. The "derivative of indocyanine green" refers to a compound in which a portion of indocyanine green is substituted by another functional group or the like while the major skeleton and functions of indocyanine green are maintained. Its excitation wavelength is in a range of 750 to 790 nm, and its fluorescence wavelength is in a range of 790 to 900 nm. Using indocyanine green or a derivative thereof whose excitation wavelength is in a range of 740 to 900 nm and whose fluorescence wavelength is in a range of 790 to 900 nm as the fluorescent dye b makes it possible to observe fluorescence-labeled cells labeled with the fluorescent probe in a living organism such as a mouse using a general-purpose in vivo fluorescence imaging apparatus without being affected by autofluorescence originating from feed or the like in the living organism such as a mouse. In addition, it is possible to observe fluorescence-labeled cells labeled with the fluorescent probe that localize in the organs in a living organism such as a mouse.

Any carrier molecule may be used as the carrier molecule as long as it can bind to the fluorescent dyes a and b and allows the different excitation wavelengths of the fluorescent dyes a and b to be maintained. Examples thereof include polymers such as inorganic polymers (e.g., polysilane, polygermane, polystannane, polysiloxane, polysilsesquioxane, polysilazane, polyborazirene, and polyphosphazene) and organic polymers (e.g., polypyrrole, polyethylene glycol, and polysaccharides). Fluorescent dyes can be selected as appropriate and used as the fluorescent dyes a and b such that the fluorescent dye b is not excited by the fluorescence from the fluorescent dye a. It is preferable that one of the fluorescent dyes a and b is incorporated into the main chain structure of the polymer serving as the carrier molecule, and the other is bound to a functional group that is present on the surface of the carrier. With such a structure, FRET is less likely to occur. It is preferable that the carrier molecule is polysiloxane (also referred to as "polymer having siloxane bonds" hereinafter) from the viewpoint of FRET not occurring between the fluorescent dyes a and b. For example, polysiloxane is formed through hydrolysis and polycondensation of silane compounds as described later.

It is preferable to use the fluorescent probe of the present invention as a cell labeling fluorescent probe. When used as a cell labeling fluorescent probe, the fluorescent probe of the present invention may be specifically bound to the surface of a cell so that the cell is labeled. In this case, it is preferable that the fluorescent probe of the present invention includes a cell surface binding substance capable of binding to a substance for specific recognition of the cell surface. The fluorescent probe specifically binds to the cell surface via a cell surface binding substance and can thus be used as a cell labeling fluorescent probe. The "substance for specific recognition of the cell surface" refers to a protein, a lipid, a sugar chain, and/or a nucleic acid that is present on the surface of a specific cell. For example, a folic acid receptor, a transferrin receptor, an antigen, and the like that are specific to cancer cells are present on the surfaces of cancer cells. Cancer cells can be specifically labeled with a fluorescent probe to which folic acid, transferrin, an antibody, or the like is bound. A cell surface marker (membrane protein) and the like are present on the surfaces of stem cells such as iPS cells and ES cells. Stem cells such as iPS cells and ES cells can be specifically labeled with a fluorescent probe to which a molecule or the like that specifically binds to the cell surface marker of iPS cells or ES cells is bound.

The fluorescent probe of the present invention may be taken up by a cell so that the cell is labeled. Examples of such a cell include a macrophage, a dendritic cell, an immune cell, a cancer cell, and an iPS cell. Macropharges, dendritic cells, immune cells, cancer cells, iPS cells, or the like that have taken up the fluorescent probe can be used to confirm the dynamic behavior of macropharges, dendritic cells, or the like inside a body in an immune cell therapy.

The fluorescent probe of the present invention can be produced by binding, preferably covalently binding, the fluorescent dye a to the carrier molecule to form a complex of the carrier molecule and the fluorescent dye a, and then binding, preferably covalently binding, the fluorescent dye b to the surface of the nanoparticle made of the complex of the carrier molecule and the fluorescent dye a to form a complex of the carrier molecule and the fluorescent dyes a and b. The cell surface binding substance may be bound to the nanoparticle made of the complex of the carrier molecule and the fluorescent dye a simultaneously when the fluorescent dye b is bound to the nanoparticle made of the complex of the carrier molecule and the fluorescent dye a. Alternatively, the fluorescent probe of the present invention can be produced by binding, preferably covalently binding, the fluorescent dye b to the carrier molecule to form a complex of the carrier molecule and the fluorescent dye b, and then binding, preferably covalently binding, the fluorescent dye a to the nanoparticle made of the complex of the carrier molecule and the fluorescent dye b to form a complex of the carrier molecule and the fluorescent dyes a and b. The cell surface binding substance may be bound to the nanoparticle made of the complex of the carrier molecule and the fluorescent dye b simultaneously when the fluorescent dye a is bound to the nanoparticle made of the complex of the carrier molecule and the fluorescent dye b. It is preferable that the excitation wavelengths and the fluorescence wavelengths of the fluorescent dyes a and b in the complex of the carrier molecule and the fluorescent dye a, the complex of the carrier molecule and the fluorescent dye b, and the complex of the carrier molecule and the fluorescent dyes a and b change minimally before and after the fluorescent dyes a and b are bound to the carrier molecule.

In order to prevent FRET from occurring between the fluorescent dyes a and b, fluorescent dyes whose excitation wavelengths are different and one of which is not excited by the fluorescence from the other fluorescent dye are selected, and/or the arrangement of the fluorescent dyes a and b in the complex of the carrier molecule and the fluorescent dyes a and b is adjusted. When polysiloxane is used as the carrier molecule in the complex of the carrier molecule and the fluorescent dyes a and b, it is preferable that one of the fluorescent dyes a and b is incorporated into a siloxane network (the main chain of the polymer), and the other is not incorporated into the siloxane network but is bound to a functional group (a functional group that is not included in the siloxane network) on the surface of the nanoparticle made of the complex of the polysiloxane and the one fluorescent dye. With such a structure, FRET is less likely to occur between the fluorescent dyes a and b.

When the carrier molecule is polysiloxane, the fluorescent dye a is porphyrin, and the fluorescent dye b is indocyanine green, the fluorescent probe can be produced as described below, for example.

First, silane having an amino group and porphyrin having a carboxyl group are reacted to obtain silane including porphyrin in its molecule (also referred to as "porphyrin-silane" hereinafter). Specifically, silane having an amino group and porphyrin having a carboxyl group are dissolved in a solvent, and a condensing agent is added thereto to initiate an amidation reaction. An example of the solvent is N,N-dimethylformamide (DMF). An example of the condensing agent is carbodiimide. An example of carbodiimide is N,N'-dipropylcarbodiimide, but there is no particular limitation thereto. Succinimide or the like may be added in order to reduce by-products. An example of succinimide is N-hydroxysuccinimide, but there is no particular limitation thereto. The reaction temperature is preferably 20 to 150°C, and more preferably 20 to 80°C, for example, from the viewpoint of synthesis cost, but there is no particular limitation thereto. The reaction time is preferably 1 to 72 hours, and more preferably 1 to 24 hours, for example, but there is no particular limitation thereto. After the reaction, the product is collected as a precipitate through centrifugation, and porphyrin-silane can be thus obtained.

In the above-mentioned reaction, the molar ratio of the silane having an amino group to the porphyrin having a carboxyl group (silane having an amino group:porphyrin having a carboxyl group) is preferably 4:1 to 1:1, more preferably 4:1 to 2:1, and even more preferably 4:1.

Next, a complex of siloxane and porphyrin is obtained through hydrolysis and polycondensation reaction between the porphyrin-silane obtained as described above and silane having one or more functional groups (also referred to as "functional silane" hereinafter). Specifically, the porphyrin-silane and the functional silane are dissolved in a solvent, and then an alkali solution is added thereto to initiate a reaction. An example of the solvent is DMF. Examples of the alkali solution include an aqueous solution of ammonia and an aqueous solution of sodium hydroxide whose pH is 8 or higher. The reaction temperature is preferably 20 to 200°C, and more preferably 60 to 80°C, for example, from the viewpoint of synthesis cost, but there is no particular limitation thereto. The reaction time is preferably 1 to 72 hours, and more preferably 3 to 24 hours, for example, but there is no particular limitation thereto. After the reaction, the product is collected as a precipitate through centrifugation, and the complex of polysiloxane and porphyrin in which porphyrin is covalently bound to polysiloxane can be thus obtained.

In the above-mentioned reaction, the molar ratio of the porphyrin-silane to the functional silane (porphyrin-silane:functional silane) is preferably 1:2 to 1:100, more preferably 1:21 to 1:50, and even more preferably 1:30 to 1:40.

There is no particular limitation on the silane having an amino group as long as an amino group is included. For example, a compound represented by General Formula (II) below can be favorably used.

Xᵢ-Si(R^{2a})ⱼ(OR^{1a})₍₄₋ᵢ₋ⱼ₎ (II)

In General Formula (II), X represents a group represented by H₂NCₘH₂ₘ-, H₂NCₙH₂ₙ-HNCₘH₂ₘ-, or Ph-NHCₘH₂ₘ- (where Ph represents a phenyl group). In particular, a group represented by H₂NCₘH₂ₘ- or H₂NCₙH₂ₙ-HNCₘH₂ₘ- is favorable. m and n are the same or different and represent an integer of 1 to 6. m is preferably 1, 2, 3, or 4, and more preferably 1, 2, or 3. n is preferably 1, 2, 3, or 4, and more preferably 1, 2, or 3. H₂NCₘH₂ₘ-, H₂NCₙH₂ₙ-HNCₘH₂ₘ-, and Ph-NHCₘH₂ₘ- are preferably H₂N(CH₂)ₘ-, H₂N(CH₂)ₙ-HN(CH₂)ₘ-, and Ph-NH(CH₂)ₘ-, respectively.

R^{1a} and R^{2a} are the same or different and represent an alkyl group having 1 to 6 carbon atoms. The alkyl group may be a linear chain or a branched chain, and is preferably a linear chain. It is preferable that the alkyl group has 1, 2, 3, or 4 carbon atoms. Specific examples of the alkyl group having 1 to 6 carbon atoms include a methyl group, an ethyl group, an n-propyl group, an isopropyl group, an n-butyl group, an isobutyl group, a tert-butyl group, a sec-butyl group, an n-pentyl group, a 1-ethylpropyl group, an isopentyl group, a neopentyl group, an n-hexyl group, a 1,2,2-trimethylpropyl group, a 3,3-dimethylbutyl group, a 2-ethylbutyl group, an isohexyl group, and a 3-methylpentyl group. In particular, a methyl group, an ethyl group, an n-propyl group, an isopropyl group, an n-butyl group, and an isobutyl group are preferable.

i represents 1 or 2, and j represents 0 or 1 (it should be noted that a relationship (4-i-j)≥2 is satisfied). That is, (i,j) represents (1,0), (1,1) or (2,0).

Examples of the compound represented by General Formula (II) include 3-aminopropyltriethoxysilane (APTES), 3-(2-aminoethylamino)propyldimethoxymethylsilane, 3-(2-aminoethylamino)propyltriethoxysilane, 3-(2-aminoethylamino)propyltrimethoxysilane, 3-aminopropyldiethoxymethylsilane, 3-aminopropyltrimethoxysilane, and trimethoxy[3-(phenylamino)propyl]silane. In particular, 3-aminopropyltriethoxysilane (APTES) and/or 3-aminopropyltrimethoxysilane are preferable.

For example, commercially available compounds manufactured by Tokyo Chemical Industry Co., Ltd. can be used as the above-described silane having an amino group. The silanes having an amino group can be used alone or in combination of two or more.

There is no particular limitation on the functional silane as long as one or more functional groups are included. The functional silane may be monofunctional silane having one functional group or polyfunctional silane having two or more functional groups. A compound represented by General Formula (III):

Yₚ-Si(R^{2c})_{q}(OR^{1c})_{(4-p-q)} (III)

can be favorably used as the silane having a functional group, for example.

In General Formula (III) above, Y represents a group represented by CH₂=CH-, a group represented by CH₂=CHCH₂-, a group including alkene, a group including thiol, a group including disulfide, a group including amine, a group including ester, a group including amide, a group including carboxylic acid, a group including urea, a group including thiourea, a group represented by OCNCH₂CH₂-, a group represented by ClC_{α}H_{2α}-, a group represented by HSC_{β}H_{2β}-, a group represented by CF₃C_{γ}F_{2γ}-C_{δ}H_{2δ}-, a group represented by CH₂=C(CH₃)COOC_{ε}H_{2ε}-, a group represented by CH₂=CHCOOC_{ζ}H_{2ζ}-, a group represented by HN-CONH-C_{η}H_{2η}-, a group represented by Chemical Formula (a) below, a group represented by Chemical Formula (b) below, an alkyl group having 1 to 18 carbon atoms, or a phenyl group.

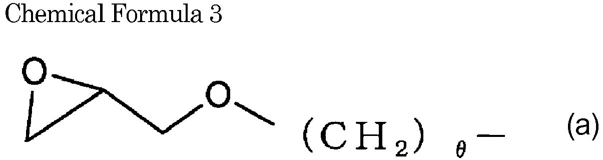

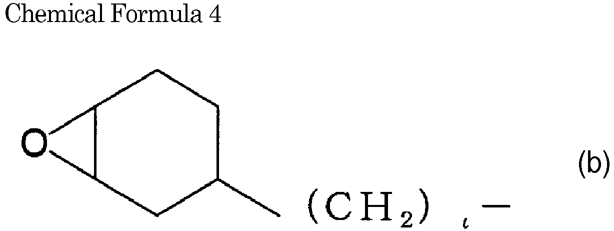

In the description above, α, β, δ, ε, ζ, η, θ, and ι independently represent an integer of 1 to 6, and preferably an integer of 1, 2, 3, or 4. γ represents an integer of 0 to 8, and preferably an integer of 0, 1, 2, 3, 4, 5, 6, or 7. The alkyl group having 1 to 18 carbon atoms preferably has 1 to 12 carbon atoms, and more preferably 1, 2, 3, 4, 5, 6, 7, or 8 carbon atoms. The alkyl group having 1 to 18 carbon atoms may be a linear chain or a branched chain, and is preferably a linear chain. ClC_{α}H_{2α}-, HSC_{β}H_{2β}-, CF₃C_{γ}F_{2γ}-C_{δ}H_{2δ}-, CH₂=C(CH₃)COOC_{ε}H_{2ε}-, CH₂=CHCOOC_{ζ}H_{2ζ}-, and HN-CONH-C_{η}H_{2η}- above are preferably Cl(CH₂)_{α}-, HS(CH₂)_{β}-, CF₃(CF₂)_{γ}-(CH₂)_{δ}-, CH₂=C(CH₃)COO(CH₂)_{ε}-, CH₂=CHCOO(CH₂)_{ζ}-, and HN-CONH-(CH₂)_{η}-, respectively.

R^{1c} represents an alkyl group having 1 to 6 carbon atoms or -(CH₂)_{τ}-OCH₃, and R^{2c} represents an alkyl group having 1 to 6 carbon atoms or a phenyl group. R^{1c} and R^{2c} may be the same or different. In both R^{1c} and R^{2c}, the alkyl group having 1 to 6 carbon atoms may be a linear chain or a branched chain, and is preferably a linear chain. An alkyl group having 1, 2, 3, or 4 carbon atoms is preferable. Specific examples of the alkyl group having 1 to 6 carbon atoms include a methyl group, an ethyl group, an n-propyl group, an isopropyl group, an n-butyl group, an isobutyl group, a tert-butyl group, a sec-butyl group, an n-pentyl group, a 1-ethylpropyl group, an isopentyl group, a neopentyl group, an n-hexyl group, a 1,2,2-trimethylpropyl group, a 3,3-dimethylbutyl group, a 2-ethylbutyl group, an isohexyl group, and a 3-methylpentyl group. In particular, a methyl group, an ethyl group, an n-propyl group, an isopropyl group, an n-butyl group, and an isobutyl group are preferable. τ represents an integer of 1 to 4 (preferably 1, 2, or 3).

p represents 1, 2, or 3, and q represents 0, 1, or 2 (it should be noted that a relationship (4-p-q)≥1 is satisfied). That is, (p,q) represents (1,0), (1,1), (1,2), (2,0), (2,1) or (3,0).

A compound represented by General Formula (IV) can also be favorably used as the functional silane, for example.

Z-SiCl₃ (IV)

In General Formula (IV) above, Z represents a group represented by CH₂=CH-, a group represented by CH₂=CHCH₂-, a group including alkene, a group including thiol, a group including disulfide, a group including amine, a group including ester, a group including amide, a group including carboxylic acid, a group including urea, a group including thiourea, a group represented by ClC_{κ}H_{2κ}-, a group represented by CF₃C_{λ}F_{2λ}-C_{ξ}H_{2ξ}-, an alkyl group having 1 to 18 carbon atoms, a phenyl group, or a cyclohexyl group. κ and ξ independently represent an integer of 1 to 6, and preferably an integer of 1, 2, 3, or 4. λ represents an integer of 0 to 8, and preferably an integer of 0, 1, 2, 3, 4, 5, 6, or 7. The alkyl group having 1 to 18 carbon atoms preferably has 1 to 12 carbon atoms, and more preferably 1, 2, 3, 4, 5, 6, 7, or 8 carbon atoms. The alkyl group having 1 to 18 carbon atoms may be a linear chain or a branched chain, and is preferably a linear chain. ClC_{κ}H_{2κ}- and CF₃C_{λ}F_{2λ}-C_{ξ}H_{2ξ}- above are preferably Cl(CH₂)_{κ}- and CF₃(CF₂)_{λ}-(CH₂)_{ξ}-, respectively.

An N-[2-(N-vinylbenzylamino)ethyl]-3-aminopropyltrimethoxysilane hydrochloride may also be used as the polyfunctional silane.

Specific examples of the compound represented by General Formula (III) include tetramethoxysilane, tetraethoxysilane, tetrapropoxysilane, tetrabutoxysilane, tetraisopropoxysilane, allyltriethoxysilane, allyltrimethoxysilane, diethoxymethylvinylsilane, dimethoxymethylvinylsilane, triethoxyvinylsilane, vinyltrimethoxysilane, vinyltris(2-methoxyethoxy)silane, (chloromethyl)triethoxysilane, 3-chloropropyldimethoxymethylsilane, 3-chloropropyltriethoxysilane, 2-(3,4-epoxycyclohexyl)ethyltrimethoxysilane, 3-mercaptopropyl(dimethoxy)methylsilane, (3-mercaptopropyl)triethoxysilane, (3-mercaptopropyl)trimethoxysilane, 3-(triethoxysilyl)propyl isocyanate, 3-(trimethoxysilyl)propyl methacrylate, triethoxy-1H,1H,2H,2H-tridecafluoro-n-octylsilane, 3-(trimethoxysilyl)propyl acrylate, 3-trimethoxysilylpropylchloride, 1-[3-(trimethoxysilyl)propyl]urea, trimethoxy(3,3,3-trifluoropropyl)silane, 3-ureidepropyltriethoxysilane, diethoxy(3-glycidyloxypropyl)methylsilane, 3-glycidyloxypropyl(dimethoxy)methylsilane, 3-glycidyloxypropyltrimethoxysilane, 2-cyanoethyltriethoxysilane, diacetoxydimethylsilane, diethoxydimethylsilane, dimethoxydimethylsilane, dimethoxydiphenylsilane, dimethoxymethylphenylsilane, dodecyltriethoxysilane, hexyltrimethoxysilane, octadecyltriethoxysilane, octadecyltrimethoxysilane, n-octyltriethoxysilane, pentyltriethoxysilane, triacetoxymethylsilane, triethoxyethylsilane, trimethoxy(methyl)silane, trimethoxyphenylsilane, and trimethoxy(propyl)silane. In particular, 3-mercaptopropyl(dimethoxy)methylsilane, (3-mercaptopropyl)triethoxysilane, (3-mercaptopropyl)trimethoxysilane, and the like are preferable.

Specific examples of the compound represented by General Formula (IV) include allyltrichlorosilane, trichlorovinylsilane, 3-chloropropyltrichlorosilane, trichloro(1H,1H,2H,2H-heptadecafluorodecyl)silane, trichloro(1H,1H,2H,2H-tridecafluoro-n-octyl)silane, butyltrichlorosilane, cyclohexyltrichlorosilane, decyltrichlorosilane, dodecyltrichlorosilane, ethyltrichlorosilane, n-octyltrichlorosilane, phenyltrichlorosilane, trichloro-2-cyanoethylsilane, trichlorohexylsilane, trichloro(methyl)silane, trichlorooctadecylsilane, trichloro(propyl)silane, and trichlorotetradecylsilane.

For example, commercially available compounds manufactured by Tokyo Chemical Industry Co., Ltd. can be used as the above-described functional silane. The functional silanes can be used alone or in combination of two or more.

Next, a complex of polysiloxane, porphyrin, and indocyanine green is formed by reacting indocyanine green with the nanoparticle made of the complex of polysiloxane and porphyrin. Specifically, an aqueous solution of the indocyanine green is added to an aqueous solution of the nanoparticles made of the complex of polysiloxane and porphyrin, and the resultant mixture is stirred at a temperature of 4 to 50°C for 1 to 24 hours and reacted. The indocyanine green is covalently bound to a functional group on the surface of the nanoparticle made of the complex of polysiloxane and porphyrin to form the complex of polysiloxane, porphyrin, and indocyanine green. Indocyanine green modified with a functional group capable of binding to the functional group present on the surface of the nanoparticle made of the complex of polysiloxane and porphyrin is used as the indocyanine green. For example, when the functional silane used to form the nanoparticle made of the complex of polysiloxane and porphyrin is a silane having a thiol group such as 3-mercaptopropyl(dimethoxy)methylsilane, (3-mercaptopropyl)triethoxysilane, or (3-mercaptopropyl)trimethoxysilane, indocyanine green to which a maleimide group is bound can be used. It should be noted that a cell surface binding substance such as folic acid may be bound to the nanoparticle made of the complex of polysiloxane and porphyrin simultaneously when indocyanine green is reacted with the nanoparticle made of the complex of polysiloxane and porphyrin to bind the indocyanine green to the nanoparticle made of the complex of polysiloxane and porphyrin. A compound obtained by binding a maleimide group to one terminus of polyethylene glycol (PEG) and folic acid to the other terminus thereof can be used as the folic acid.

In the above-mentioned reaction, the molar ratio of the functional group of the nanoparticle made of the complex of polysiloxane and porphyrin that is covalently bound to indocyanine green to the functional group with which the indocyanine green is modified and that is covalently bound to the nanoparticle made of the complex of polysiloxane and porphyrin (the functional group of the nanoparticle made of the complex of polysiloxane and porphyrin that is covalently bound to indocyanine green:the functional group with which the indocyanine green is modified) is preferably 1:1 to 3:1, more preferably 1:1 to 2:1, and even more preferably 1:1.

When TCPP is used as the porphyrin, it is preferable that the carboxyl group of TCPP forms an amide bond in the complex of polysiloxane and porphyrin, and it is more preferable that TCPP is incorporated into the siloxane network (main chain of polysiloxane) by an amide bond. It is preferable that the indocyanine green is bound to the functional group (functional group of the functional silane included in the nanoparticle made of polysiloxane and porphyrin) on the surface of the nanoparticle made of the complex of polysiloxane and porphyrin. With such a structure, FRET does not occur between the porphyrin and the indocyanine. In the present invention, the structure of a complex such as the complex of polysiloxane and porphyrin or the fluorescent probe can be analyzed using Fourier transform infrared spectrophotometry as described later.

The fluorescent probe preferably includes the fluorescent dye a in an amount of 1 mass% or more and the fluorescent dye b in an amount of 0.1 mass% or more, and more preferably the fluorescent dye a in an amount of 5 to 90 mass% and the fluorescent dye b in an amount of 1 to 10 mass%, from the viewpoint of being suitable for both in vitro fluorescence imaging and in vivo fluorescence imaging, but there is no particular limitation thereto. When the fluorescent dye a is porphyrin, the fluorescent dye b is indocyanine green, and the carrier molecule is polysiloxane, the fluorescent probe preferably includes four pyrrole ring structure moieties (porphine) in porphyrin in an amount of 1 mass% or more and the indocyanine green in an amount of 0.1 mass% or more. It is more preferable that the content of the four pyrrole ring structure moieties (porphine) in porphyrin is 5 to 90 mass%, and the content of the indocyanine green is 1 to 10 mass%. As described later, in the present invention, analyzing a complex such as the complex of polysiloxane and porphyrin, or a fluorescent probe through thermogravimetric-differential thermal analysis makes it possible to measure the content of a fluorescent dye such as porphyrin and a carrier molecule such as silica polymer.

The fluorescent probe preferably has an average particle diameter of 3 to 250 nm, and more preferably 10 to 80 nm, from the viewpoint that cells are easy to label, but there is no particular limitation thereto. In the present invention, the average particle diameter is measured through dynamic light scattering.

### Method for detecting fluorescence

A method for detecting fluorescence of the present invention is used to observe target cells. Specifically, the method for detecting fluorescence includes a step of labeling target cells with a fluorescent probe, and a step of irradiating the target cells labeled with the fluorescent probe with excitation light and observing fluorescence from the fluorescent probe. The above-described fluorescent probe of the present invention is used as the fluorescent probe.

Target cells can be labeled by binding the fluorescent probe to the cell surface or causing cells to take up the fluorescent probe. When the fluorescent probe is bound to the cell surface, the fluorescent probe includes a cell surface binding substance. For example, when the fluorescent probe includes folic acid, cancer cells such as HeLa S3 cells derived from human cervical cancer and HCT116 cells derived from human large intestine cancer can be labeled by binding the fluorescent probe to the surfaces of these cancer cells. Specifically, culturing cells in a cell culture medium containing the fluorescent probe makes it possible to label the cells with the fluorescent probe. Examples of the target cells include stem cells such as iPS cells and ES cells, and disease-related cells such as cancer cells and cirrhotic cells.

When the target cells labeled with the fluorescent probe are irradiated with excitation light that excites the fluorescent dye, the fluorescence from the fluorescent probe can be observed through in vitro fluorescence imaging using an in vitro fluorescence imaging apparatus such as a fluorescence microscope or a flow cytometer. For example, the fluorescent dye a is excited by an excitation light with a wavelength in a range of 350 to 650 nm, and a fluorescence with a wavelength in a range of 400 to 800 nm is observed. It is preferable that the fluorescent dye a is excited by an excitation light with a wavelength in a range of 380 to 580 nm, and a fluorescence with a wavelength in a range of 450 to 680 nm is observed.

When the target cells labeled with the fluorescent probe are transplanted into a living organism (excluding a human), and the target cells labeled with the fluorescent probe in the living organism are irradiated with excitation light that excites the fluorescent dye b, the fluorescence from the fluorescent probe can be observed through in vivo fluorescence imaging using an in vivo fluorescence imaging apparatus. For example, the fluorescent dye b is excited by an excitation light with a wavelength in a range of 600 to 850 nm, and a fluorescence with a wavelength in a range of 650 to 920 nm is observed. It is preferable that the fluorescent dye b is excited by an excitation light with a wavelength in a range of 630 to 790 nm, and a fluorescence with a wavelength in a range of 680 to 910 nm is observed. In the present invention, there is no particular limitation on the living organism as long as the living organism is an animal capable of undergoing fluorescence observation. Mammals are preferable. Mammals belonging to Rodentia such as mice and rats are particularly preferable. It should be noted that the target cells labeled with the fluorescent probe can be transplanted into a living organism such as a mouse as described later.

Using the fluorescent probe of the present invention makes it possible to observe the target cells labeled with the fluorescent probe both in vitro and in vivo.

### Method for using fluorescent probe

A method for using a fluorescent probe of the present invention includes a step of fluorescently labeling cells with a fluorescent probe, a step of screening the fluorescence-labeled cells labeled with the fluorescent probe through observation using flow cytometer or a fluorescence microscope, a step of transplanting the screened fluorescence-labeled cells into a living organism (excluding a human), and a step of observing the fluorescence-labeled cells in the living organism using an in vivo fluorescence imaging apparatus.

First, cells are fluorescently labeled with the fluorescent probe. Cells can be labeled by binding the fluorescent probe to the cell surface or causing cells to take up the fluorescent probe. When the fluorescent probe is bound to the cell surface, the fluorescent probe includes a cell surface binding substance. For example, when the fluorescent probe includes folic acid, cancer cells such as HeLa S3 cells derived from human cervical cancer and HCT116 cells derived from human large intestine cancer can be labeled by binding the fluorescent probe to the surfaces of these cancer cells. Specifically, culturing cells in a cell culture medium containing the fluorescent probe makes it possible to label the cells with the fluorescent probe. Examples of the cells include stem cells such as iPS cells and ES cells, and disease-related cells such as cancer cells and cirrhotic cells.

Next, the fluorescence-labeled cells labeled with the fluorescent probe are screened using a flow cytometer. The fluorescence-labeled cells are confirmed and screened using a flow cytometer or a fluorescence microscope by detecting the fluorescence originating from the fluorescent dye a. Specifically, the fluorescence-labeled cells that have been fluorescently labeled with the fluorescent probe can be screened by supplying the cells that have been subjected to a fluorescence labeling operation using a fluorescent probe to a flow cytometer, allowing the fluorescent dye a to be excited by an excitation light with a wavelength that is an excitation wavelength of the fluorescent dye a, and detecting the fluorescence from the excited fluorescent dye a. Observation using a flow cytometer can be performed provided that the excitation wavelength is in a range of 350 to 640 nm and the fluorescence wavelength is in a range of 630 to 780 nm, for example. Alternatively, the fluorescence-labeled cells that have been fluorescently labeled with the fluorescent probe can be screened by supplying the cells that have been subjected to a fluorescence labeling operation using a fluorescent probe to a fluorescence microscope, allowing the fluorescent dye a to be excited by an excitation light with a wavelength that is an excitation wavelength of the fluorescent dye a, and detecting the fluorescence from the excited fluorescent dye a. Observation using a fluorescence microscope can be performed provided that the excitation wavelength is in a range of 350 to 640 nm and the fluorescence wavelength is in a range of 630 to 780 nm, for example. Conventionally, cells that have been subjected to a fluorescence labeling operation using a fluorescent probe are transplanted into a living organism such as a mouse as they are, but using the fluorescent probe of the present invention makes it possible to screen only fluorescence-labeled cells that are fluorescently labeled with the fluorescent probe using a flow cytometer and transplant the screened fluorescence-labeled cells into a living organism such as a mouse.

Next, the screened fluorescence-labeled cells are transplanted into a living organism (excluding a human). The screened fluorescence-labeled cells can be transplanted into a living organism through subcutaneous administration, intravenous administration, intraperitoneal administration, or the like, for example. In a case of using a mouse, for example, the fluorescence-labeled cells are transplanted into a mouse by suspending the fluorescence-labeled cells in phosphate buffered saline and injecting the suspension of the fluorescence-labeled cells into the caudal vein of the mouse using a syringe.

Next, the fluorescence-labeled cells in the living organism are observed using an in vivo fluorescence imaging apparatus. There is no particular limitation on the in vivo fluorescence imaging apparatus, and an in vivo fluorescence imaging apparatus "KURAVIC KV-700" manufactured by Kurabo Industries Ltd., IVIS Imaging System, Maestro (trademark) manufactured by PerkinElmer Co., Ltd, or the like can be used. The fluorescence-labeled cells in the living organism are observed using an in vivo fluorescence imaging apparatus by detecting the fluorescence originating from the fluorescent dye b. Specifically, the fluorescent dye b is excited by an excitation light with a wavelength that is an excitation wavelength of the fluorescent dye b, and the fluorescence from the excited fluorescent dye b is detected. The fluorescence-labeled cells in the living organism can be observed using an in vivo fluorescence imaging apparatus provided that the excitation wavelength is in a range of 650 to 760 nm and the fluorescence wavelength is in a range of 760 to 850 nm, for example. Observing the fluorescence-labeled cells in the living organism using an in vivo fluorescence imaging apparatus over time makes it possible to obtain information about positions at which cells such as stem cells including iPS cells, ES cells, and the like and disease-related cells including cancer cells, cirrhotic cells, and the like that have been transplanted into the living organism such as a mouse differentiate, grow, and metastasize after being transplanted.

Conventionally, after being labeled with a fluorescent probe, cells are transplanted into a living organism such as a mouse without confirming whether or not the cells are actually labeled with the fluorescent probe. However, using the fluorescent probe of the present invention makes it possible to screen only fluorescence-labeled cells labeled with the fluorescent probe using a flow cytometer, transplant only the fluorescence-labeled cells into a living organism such as a mouse, and observe the progression of the transplanted fluorescence-labeled cells in the living organism using an in vivo fluorescence imaging apparatus.

### Examples

Hereinafter, the present invention will be specifically described by way of examples. It should be noted that the present invention is not limited to the following examples.

### Reagents

All of tetrakis(4-carboxyphenyl)porphyrin (TCPP), (3-mercaptopropyl)trimethoxysilane (MPTMS), N,N-dimethylformamide (DMF), indocyanine green to which a maleimide group is bound (molecular weight: 853; also referred to simply as "ICG-Mal" hereinafter), and polyethylene glycol (PEG) in which folic acid and a maleimide group are respectively bound to both of the termini (molecular weight: 3400; also referred to simply as "FA-PEG-Mal" hereinafter) were obtained from Tokyo Chemical Industry Co., Ltd. The following are the structural formulae of MPTMS, ICG-Mal, and FA-PEG-Mal.

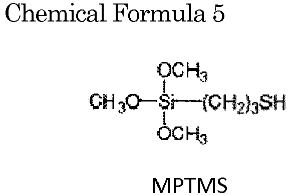

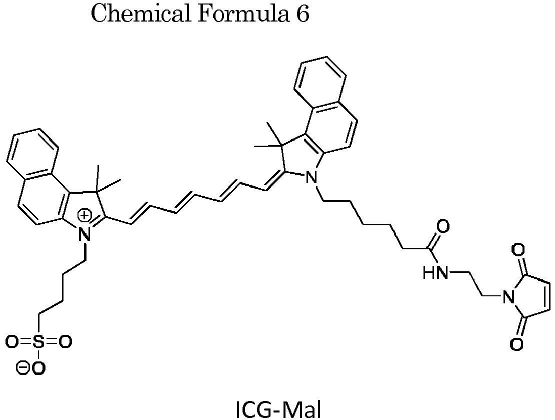

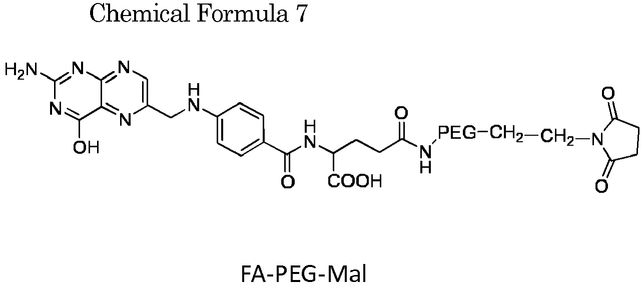

### Example 1

### Production of fluorescent probe

### Production of complex of carrier molecule and fluorescent dye a

(1) APTES (462 µmol) was added to a DMF solution of TCPP (3.8 mM: 32 mL) obtained by dissolving TCPP in DMF, and then N,N'-dipropylcarbodiimide (480 µmol) and N-hydroxysuccinimide (480 µmol) were added thereto. The resultant mixture was stirred at 50°C for 24 hours.
(2) Next, 200 µL (porphyrin-silane: 0.75 µmol) of the DMF solution of porphyrin-silane obtained as described above was mixed with 5 µl (0.027 mmol) of MPTMS. To the obtained mixed solution, 500 µL of DMF and 300 µL of an aqueous solution of ammonia (concentration: 28 mass%, pH 11) were added, and a reaction was carried out at 80°C for 24 hours.
(3) After 24 hours, the product was collected as a precipitate through centrifugation (15000 rpm for 20 minutes). Then, the product was washed with water and ethanol several times, and was finally dispersed in 1 mL of water.
(4) The obtained substance was a complex of polysiloxane and porphyrin in which the polysiloxane serves as a carrier molecule and the porphyrin serves as a fluorescent dye a, and the porphyrin is covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "PPS HNPs" hereinafter) with an average particle diameter of about 40 nm.

### Step of binding fluorescent dye b to complex of carrier molecule and fluorescent dye a

(1) To 1 mL of the aqueous dispersion of the PPS HNPs obtained as described above, 63 µl of an aqueous solution of ICG-Mal (concentration: 2 mg/mL, ICG-Mal: 1.48×10⁻⁴ mmol) was added. The resultant mixture was stirred at 30°C for 3 hours to carry out a reaction.
(2) After the reaction, the product was collected as a precipitate through centrifugation (15000 rpm for 20 minutes). Then, the product was washed with water several times, and was finally dispersed in 1 mL of water.
(3) The obtained substance was a complex (fluorescent probe) in which the polysiloxane serves as a carrier molecule, the porphyrin serves as a fluorescent dye a, and the indocyanine green serves as a fluorescent dye b, and the porphyrin and the indocyanine green were covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "ICG-PPS HNPs" hereinafter) with an average particle diameter of about 50 nm.

### Example 2

### Production of fluorescent probe

### Production of complex of carrier molecule and fluorescent dye a

PPS HNPs was obtained in the same manner as in Example 1.

### Step of binding fluorescent dye b and cell surface binding substance to carrier molecule

(1) To 1 mL of the aqueous dispersion of the PPS HNPs obtained as described above, 251 µl of an aqueous solution of FA-PEG-Mal (concentration: 2 mg/mL, FA-PEG-Mal: 1.48×10⁻⁴ mmol) and 63 µl of an aqueous solution of ICG-Mal (concentration: 2 mg/mL, ICG-Mal: 1.48×10⁻⁴ mmol) were added. The resultant mixture was stirred at 30°C for 3 hours.
(2) After the reaction, the product was collected as a precipitate through centrifugation (15000 rpm for 20 minutes). Then, the product was washed with water several times, and was finally dispersed in 1 mL of water.
(3) The obtained substance was a complex (fluorescent probe) in which the polysiloxane serves as a carrier molecule, the porphyrin serves as a fluorescent dye a, the indocyanine green serves as a fluorescent dye b, and the folic acid serves as a cell surface binding substance, and the porphyrin, the indocyanin green, and the folic acid are covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "FA-PEG/ICG-PPS HNPs" hereinafter) with an average particle diameter of about 65 nm.

### Confirmation through Fourier transform infrared spectrophotometry

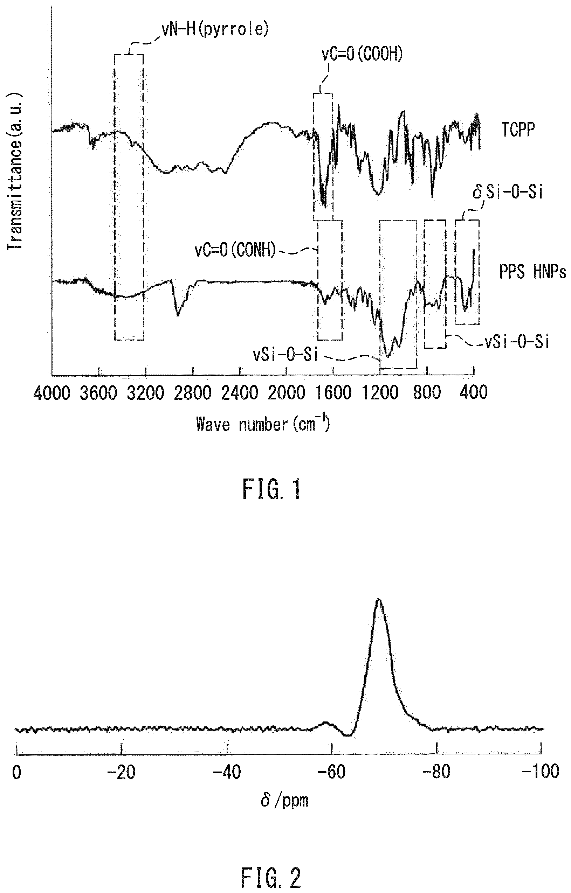

The PPS HNPs obtained in Examples 1 and 2 and the TCPP used as a raw material were analyzed using a Fourier transform infrared spectrophotometer ("NEXUS470" manufactured by Nicolet). FIG. 1 shows FT-IR spectra of the PPS HNPs and the TCPP. It was found from the FT-IR spectra shown in FIG. 1 that both of them exhibited a peak originating from a pyrrole ring at 3430 to 3250 cm⁻¹. Since a peak originating from carboxylic acid (carboxyl group) of the TCPP at 1800 to 1640 cm⁻¹ disappeared and a peak originating from amide at 1740 to 1620 cm⁻¹ appeared in the FT-IR spectrum of the PPS HNPs, it was confirmed that the complex of polysiloxane and porphyrin in which a carboxyl group of the TCPP forms an amide bond and is thus covalently bound to the siloxane (that is, the complex of polysiloxane and porphyrin formed through hydrolysis and condensation of the porphyrin-silane and the MPTMS) was reliably synthesized. In addition, peaks originated from siloxane bonds appeared at 1260 to 1010 cm⁻¹ (vSi-O-Si), 870 to 740 cm⁻¹ (vSi-O-Si), and 540 to 410 cm⁻¹ (σSi-O-Si) in the FT-IR spectrum of the PPS HNPs. That is, it was confirmed that the porphyrin-silane and the MPTMS (functional silane) formed a siloxane network in PPS HNPs, and the porphyrin was incorporated into the siloxane network by an amide bond.

The structure of the PPS HNPs obtained in Examples 1 and 2 was analyzed through ²⁹Si solid-state NMR. FIG. 2 shows a ²⁹Si solid-state NMR spectrum of the PPS HNPs. It was confirmed from FIG. 2 that peaks appeared at -58 ppm and -69 ppm. It was inferred that the peak at -58 ppm originated from the T² structure represented by a chemical formula below, and the peak at -69 ppm originated from the T³ structure represented by a chemical formula below. It was confirmed from this result that a portion of the PPS HNPs was constituted by Si with the T₂ structure, but most of the MPTMS and porphyrin-silane were subjected to hydrolysis and polycondensation. That is, the porphyrin was covalently bound to the polysiloxane, and the complex was thus formed.

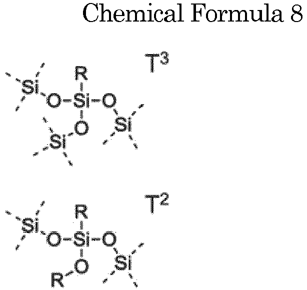

### Thermogravimetric-differential thermal analysis

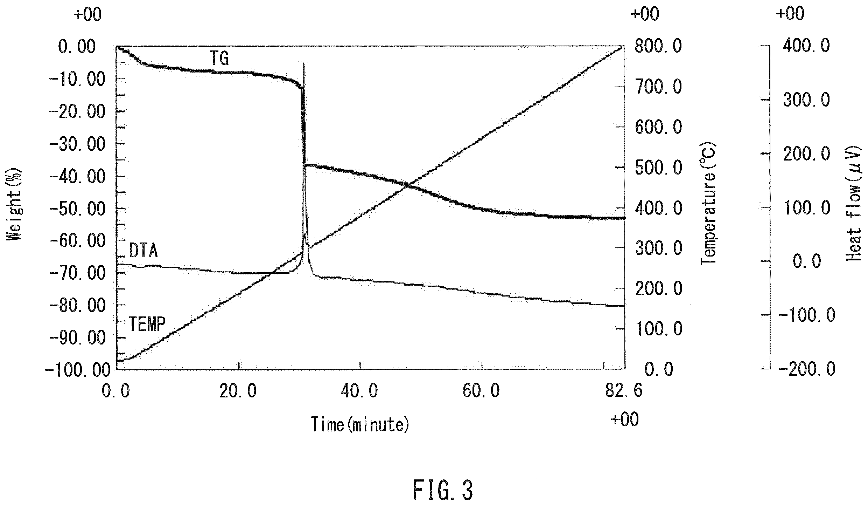

The PPS HNPs was analyzed through thermogravimetric-differential thermal analysis (TG-DTA) using a thermal analyzer (TG8120, manufactured by Rigaku Corporation). FIG. 3 shows a TG (thermogravimetric) curve and a DTA (differential thermal analysis) curve of the PPS HNPs. It is inferred from the analysis results that the weight reduction at 100°C was due to adsorbed water evaporating, the weight reduction at 300°C was due to the combustion of the aminopropyl moiety and the mercaptopropyl moiety, the weight reduction at 300 to 400°C was due to the decomposition of the pyrrole ring structure moieties (porphine) in the porphyrin, and the remaining portion was constituted by the polysiloxane moiety. Specifically, it was inferred that the PPS HNPs contained the adsorbed water in an amount of 7 wt%, the aminopropyl moiety and mercaptopropyl moiety in an amount of 29 wt%, the pyrrole ring structure moieties (porphine) in the porphyrin in an amount of 17 wt%, and the polysiloxane moiety in an amount of 47 wt%.

### Quantification of FA-PEG and ICG bound to PPS HNPs

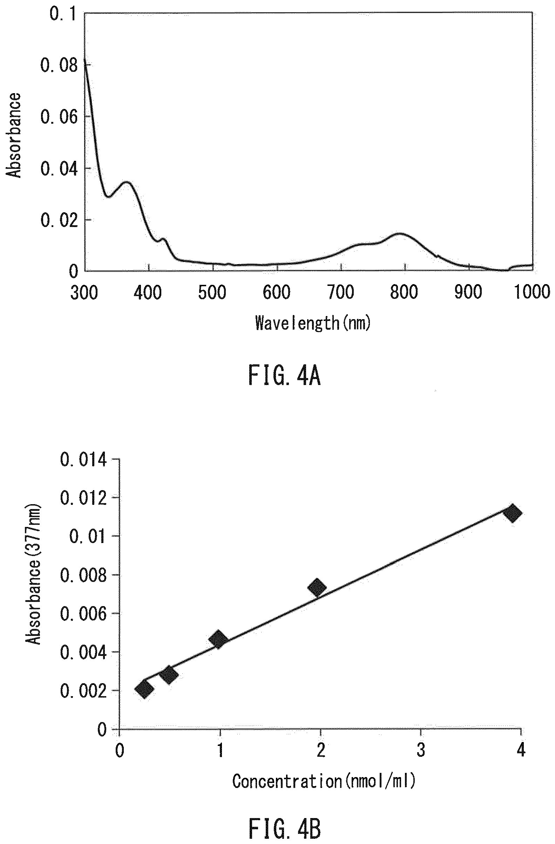

In order to determine the amounts of the FA-PEG and the ICG bound to the PPS HNPs, an absorption spectrum of the supernatant (1 mL) after the reaction was measured using a spectrophotometer ("V-570" manufactured by JASCO Corporation), and the amounts of unreacted FA-PEG-Mal and ICG-Mal were calculated. FIG. 4A shows the absorption spectrum of the supernatant. Regarding the FA-PEG-Mal, the extinction coefficient of folic acid in an aqueous solution was unknown, and therefore, a calibration curve of folic acid at a peak wavelength of 377 nm was produced, and the concentration was calculated using this calibration curve. FIG. 4B shows the calibration curve for folic acid. In FIG. 4A, the absorbance at 377 nm is 0.032, and it was thus determined that the concentration of the folic acid in the supernatant was 12 nmol/mL. The amount of the used FA-PEG-Mal was 148 nmol, and therefore, it was confirmed that 92% of the FA-PEG-Mal was reacted. Next, regarding the ICG-Mal, the concentration of the ICG was calculated based on the formula of Lambert-Beer law, namely A₈₀₀=ε₈₀₀cL using the molar extinction coefficient of the ICG in an aqueous solution at 800 nm, namely ε₈₀₀=147000 L/mol·cm. As a result, the concentration of the ICG was 0.094 nmol/mL. The amount of the used ICG-Mal was 148 nmol, and therefore, it could be estimated that 99.9% of the ICG-Mal was reacted. It was determined from these calculation results that 92% of the FA-PEG and substantially 100% of the ICG were bound to the PPS HNPs. When the amount of the ICG bound to the PPS HNPs was determined in Example 1 in the same manner, it was confirmed that substantially 100% of the ICG was bound to the PPS HNPs.

### Confirmation of absorption spectrum through spectroscopy

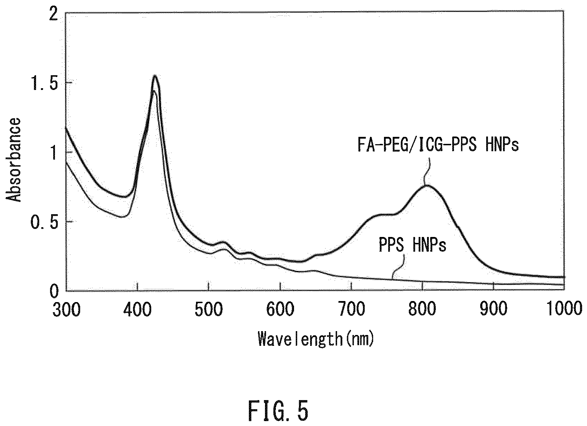

The absorption spectra of the aqueous solution of the PPS HNPs obtained in Examples 1 and 2 and the aqueous solution of the FA-PEG/ICG-PPS HNPs obtained in Example 2 were measured using a spectrophotometer ("V-570" manufactured by JASCO Corporation). FIG. 5 below shows the results. It was confirmed from the absorption spectra of the PPS HNPs and the FA-PEG/ICG-PPS HNPs shown in FIG. 5 that a peak originating from ICG appeared around a wavelength of 700 to 900 nm due to the ICG being bound to the PPS HNPs. Also, it was confirmed that a peak around 380 to 650 nm originating from porphyrin minimally changed in the ICG-PPS HNPs and the FA-PEG/ICG-PPS HNPs.

### Confirmation of wavelength characteristics of fluorescent probe

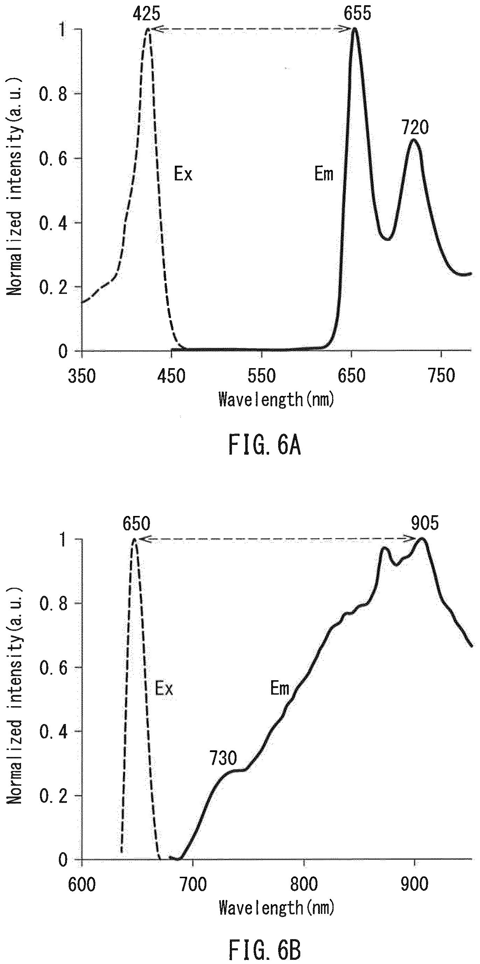

The aqueous solution of the FA-PEG/ICG-PPS HNPs obtained in Example 2 was used to analyze the excitation wavelength spectrum and the fluorescence wavelength spectrum of the FA-PEG/ICG-PPS HNPs using a fluorescence spectrophotometer FP-6600 manufactured by JASCO Corporation. FIG. 6 shows the results. FIG. 6A shows the wavelength characteristics on a short wavelength side to be used in in vitro fluorescence imaging, and FIG. 6B shows the wavelength characteristics on a long wavelength side to be used in in vivo fluorescence imaging. It was found from FIGS. 6A and 6B that Stokes shift was large on both the short wavelength side with a maximum excitation wavelength of 425 nm and a maximum fluorescence wavelength of 655 nm and the long wavelength side with a maximum excitation wavelength of 650 nm and a maximum fluorescence wavelength of 905 nm. It was confirmed that both the ICG-PPS HNPs and the FA-PEG/ICG-PPS HNPs were fluorescent probes that include two fluorescent dyes with different excitation wavelengths (porphyrin and ICG) bound to a carrier molecule (polysiloxane) and in which fluorescence resonance energy transfer does not occur between the two fluorescent dyes. The fluorescent probe can be used in both in vitro imaging and in vivo imaging. Furthermore, since the Stokes shift is large, the fluorescent probe is not disturbed by autofluorescence of a living organism in in vivo imaging and is thus expected to be very useful.

### Analysis using fluorescence microscope

### (1) Cells

RAW264.7 cells derived from a mouse macrophage, which are homologous to mouse cells, and HeLa S3 cells derived from human cervical cancer and HCT116 cells derived from human large intestine cancer, which are not homologous to mouse cells, were used. In the examples, cells and cell culture media were obtained from Sigma-Aldrich. Cell culture dishes manufactured by Thermo Fisher Scientific were used.

### (2) Fluorescent probe labeling of cells

(a) Cells (0.3×10⁵ cells/mL) were seeded into a cell culture medium (DMEM medium containing 10% FBS) in a cell culture dish (with a diameter of 35 mm) and cultured overnight.
(b) The cultured cells were transferred into a cell culture medium (DMEM medium containing 10% FBS) containing the fluorescent probe (30 µg/mL), and cultured for 24 hours. At this time, the RAW264.7 cells were transferred into a cell culture medium containing the fluorescent probe ICG-PPS HNPs, and the HeLa S3 cells and the HCT116 cells were transferred into a cell culture medium containing the fluorescent probe FA-PEG/ICG-PPS HNPs.
(c) The supernatant was removed, and the cultured cells were washed with 2 mL of phosphate buffered saline (1×PBS, pH 7.4: "D-PBS(-) (045-29795)" manufactured by Wako Pure Chemical Corporation).
(d) The supernatant was removed, and 2 mL of 4% paraformaldehyde (PFA) was added to the cultured cells. The cells were left to stand at room temperature (20±5°C) for 30 minutes.
(e) The supernatant was removed, and 1 mL of 1×PBS was added to the cultured cells. This step was repeated three times.

### (3) Observation using fluorescence microscope

The fluorescence-labeled cells labeled with the fluorescent probe were observed using a fluorescence microscope EVOS (registered trademark) FL Cell Imaging System manufactured by Life Technologies. The fluorescence-labeled cells were observed using an excitation wavelength of 422 nm and a fluorescence wavelength of 655 nm based on the wavelength characteristics of porphyrin. FIG. 7 shows the results.

FIG. 7A shows a bright field image (Bright field) of RAW264.7 cells, a fluorescence image (DAPI) of cell nuclei using a fluorescent dye DAPI (4,6-diamidino-2-phenylindole), fluorescence imaging using the ICG-PPS HNPs (fluorescent probe), and an image (Merge) obtained by merging these three images together. It was found from the bright field image shown in FIG. 7A that the cells were present. In the DAPI image, the cell nuclei stained with DAPI were confirmed. It was found from the ICG-PPS HNPs image that the cells were labeled with the fluorescent probe. It was found from the Merge image that fluorescence of the fluorescent probe was observed more frequently in the cytoplasm in the RAW264.7 cells, and the nuclei did not emit fluorescence. It was confirmed from these results that the ICG-PPS HNPs (fluorescent probe) was taken up by the cell and stayed in the cytoplasm. The RAW264.7 cells are macrophage cells, and therefore, it was thought that the fluorescent probe was taken up by the cell due to its phagocytic activity, and fluorescent probes accumulated in the cytoplasm.

FIG. 7B shows a bright field image (Bright field) of HeLa S3 cells, a fluorescence image (DAPI) of cell nuclei using a fluorescent dye DAPI, fluorescence imaging using the FA-PEG/ICG-PPS HNPs fluorescent probe, and an image (Merge) obtained by merging these three images together. It was found from the bright field image shown in FIG. 7B that the cells were present. In the DAPI image, cell nuclei stained with DAPI were confirmed. It was found from the FA-PEG/ICG-PPS HNPs fluorescent probe image that the cells were labeled with the fluorescent probe. It was found from the Merge image that the fluorescent probe emitted fluorescence from the entire HeLa S3 cell including the cell nucleus. It was revealed from these results that the fluorescent probe was bound to the cell surface, and light emission from the entire cell was thus observed. It was thought that cancer cells have a receptor to which folic acid binds on the cell surface, and the fluorescent probe (FA-PEG/ICG-PPS HNPs) was bound to the surface of the HeLa S3 cell, which is a cancer cell, via folic acid.

FIG. 7C shows a bright field image (Bright field) of HCT116 cells, a fluorescence image (DAPI) of cell nuclei using a fluorescent dye DAPI, fluorescence imaging using the FA-PEG/ICG-PPS HNPs fluorescent probe, and an image (Merge) obtained by merging these three images together. It was found from the bright field image shown in FIG. 7C that the cells were present. In the DAPI image, cell nuclei stained with DAPI were confirmed. It was found from the FA-PEG/ICG-PPS HNPs fluorescent probe image that the cells were labeled with the fluorescent probe. It was found from the Merge image that the fluorescent probe emitted fluorescence from the entire HCT116 cell including the cell nucleus. It was revealed from these results that the fluorescent probe was bound to the cell surface, and light emission from the entire cell was thus observed. It was thought that cancer cells have a receptor to which folic acid binds on the cell surface, and the fluorescent probe (FA-PEG/ICG-PPS HNPs) was bound to the surface of the HCT116 cell, which is a cancer cell, via folic acid.

### Analysis using flow cytometer

### (1) Cells

RAW264.7 cells derived from a mouse macrophage, which are homologous to mouse cells, and HeLa S3 cells derived from human cervical cancer and HCT116 cells derived from human large intestine cancer, which are not homologous to mouse cells, were used.

### (2) Fluorescent probe labeling of cells

(a) Cells were seeded into a cell culture medium (DMEM medium containing 10% FBS) in a cell culture dish (with a diameter of 35 mm) in a seeding amount shown in Table 1 below and cultured overnight.
(b) The cultured cells were transferred into a cell culture medium (DMEM medium containing 10% FBS) containing the fluorescent probe (50 pg/mL) shown in Table 1 below, and cultured for another 48 hours.
(c) The cells were collected from the cell culture dish. At this time, the HeLa S3 cells and the HCT116 cells were collected using a 0.25% trypsin/EDTA solution.
(d) After the collected solution was centrifuged at 1000 revolutions for 5 minutes, the supernatant was removed, and the cells were suspended in 400 µL of a cell culture medium (DMEM medium containing 10% FBS).
(e) After the suspension was centrifuged at 1000 revolutions for 5 minutes, the supernatant was removed, and 1 mL of 4% PFA was added to the cells. The cells were left to stand at room temperature (20±5°C) for 30 minutes.
(f) After the resuspension was centrifuged at 1000 revolutions for 5 minutes, the supernatant was removed, and 1 mL of 1×PBS was added to the cells. This step was repeated three times.

**Table 1**

| Type of cells | Origin | Fluorescent probe | Localization of fluorescent probe | Cell seeding amount (cells/mL) |
|---|---|---|---|---|
| RAW264.7 cells | Mouse | ICG-PPS HNPs | Intracellular | 2×10⁵ |
| HeLa S3 cells | Human | FA-PEG/ICG-PPS HNPs | On cell surface | 2×10⁵ |
| HCT116 cells | Human | FA-PEG/ICG-PPS HNPs | On cell surface | 5×10⁵ |

### Observation using flow cytometer

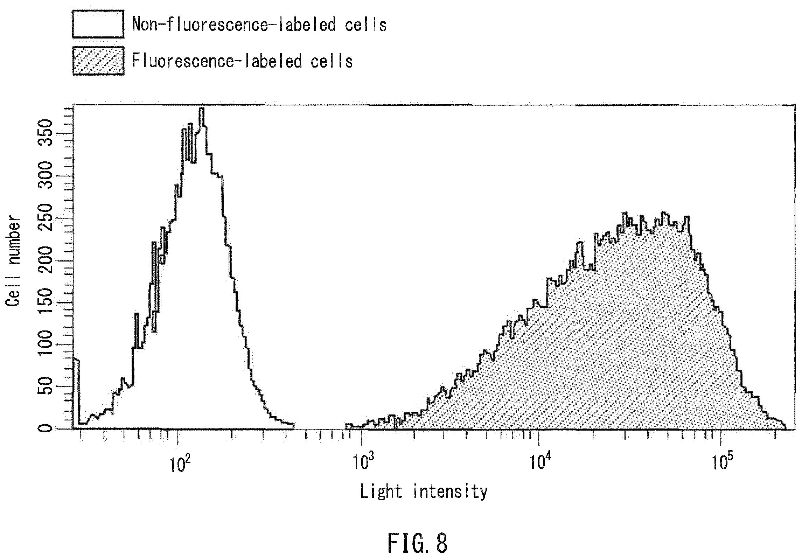

The cells labeled with the fluorescent probe were observed using a flow cytometer LSR Fortessa X-20 manufactured by BD. The observation was performed using an excitation wavelength of 405 nm and a fluorescence wavelength of 670 nm. The results are shown in FIGS. 8 (RAW264.7 cells), 9 (HeLa S3 cells), and 10 (HCT116 cells).

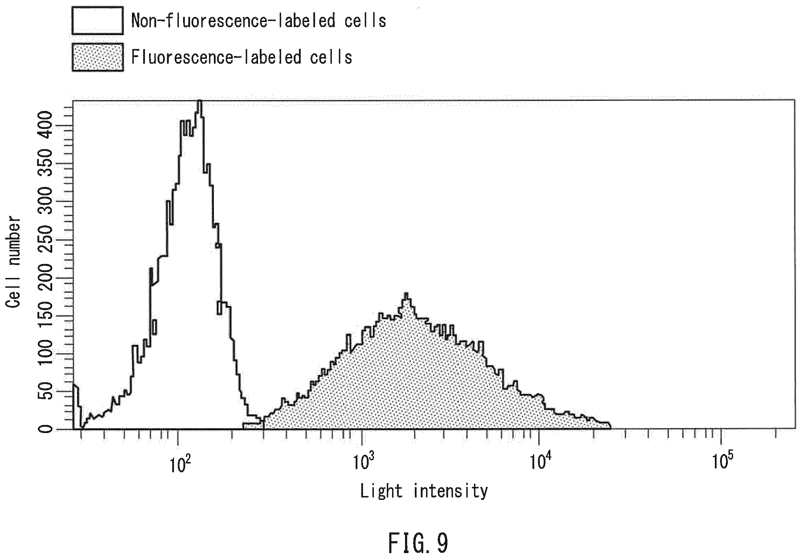

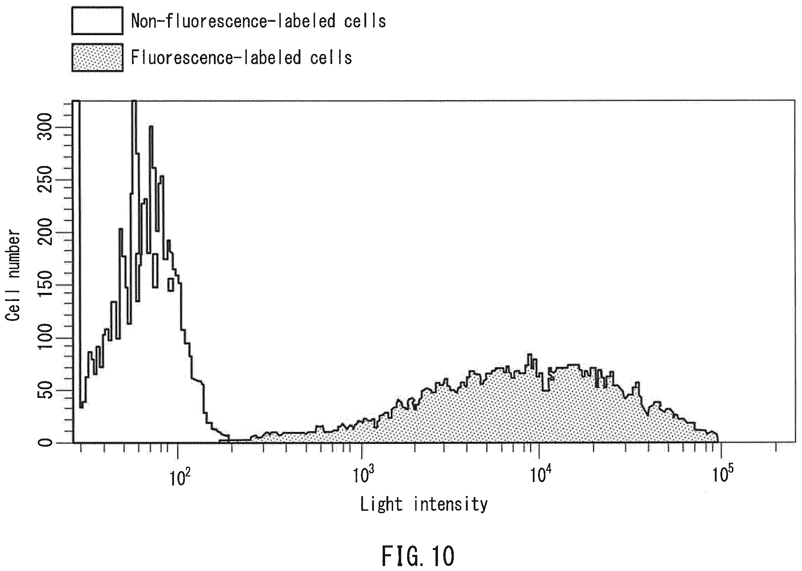

As is clear from FIGS. 8 to 10, in all of the cell samples, the fluorescence-labeled cells and the non-fluorescence-labeled cells were separately observed. The light intensities were different by a factor of about 100 to 10000 times. After the cells were labeled with the fluorescent probe, the fluorescence-labeled cells and the non-fluorescence-labeled cells could be clearly distinguished and separated. Using a flow cytometer makes it possible to screen only the fluorescence-labeled cells labeled with the fluorescent probe, transplanting only the fluorescence-labeled cells into a living organism such as a mouse, and more precisely confirm the localization of the fluorescence-labeled cells in the living organism such as a mouse using an in vivo fluorescence imaging apparatus.

### Transplantation of fluorescence-labeled cells into mouse

(1) Cells
   RAW264.7 cells and HeLa S3 cells were used.
(2) Fluorescent probe labeling of cells
   (a) Cells (1×10⁶ cells/mL) were seeded into a cell culture medium (DMEM medium containing 10% FBS) in a cell culture dish (with a diameter of 60 mm) and cultured overnight.
   (b) The cultured cells were transferred into a cell culture medium (DMEM medium containing 10% FBS) containing the fluorescent probe (30 µg/mL), and cultured for 24 hours. At this time, the RAW264.7 cells were transferred into a cell culture medium containing the fluorescent probe ICG-PPS HNPs, and the HeLa S3 cells were transferred into a cell culture medium containing the fluorescent probe FA-PEG/ICG-PPS HNPs.
   (c) The cells were collected from the cell culture dish. At this time, the HeLa S3 cells were collected using a trypsin/EDTA solution.
   (d) After the collected solution was centrifuged at 1000 revolutions for 5 minutes, the supernatant was removed, and the cells were suspended in 1000 µL of a serum-free culture medium (DMEM medium).
   (e) After the suspension was centrifuged at 1000 revolutions for 5 minutes, the supernatant was removed, and 1000 µL of a serum-containing culture medium (DMEM medium containing 10% FBS) was added to the cells and the cells were suspended.
   (f) In 100 µL of 1×PBS, 1×10⁶ cells were suspended, and the suspension was injected into the caudal vein of a mouse (KSN/Slc, male, 6 weeks old, obtained from Japan SLC, Inc.) using a syringe. The number of cells was measured using a hemocytometer.
(3) Observation of fluorescence-labeled cells labeled with fluorescent probe in mouse body

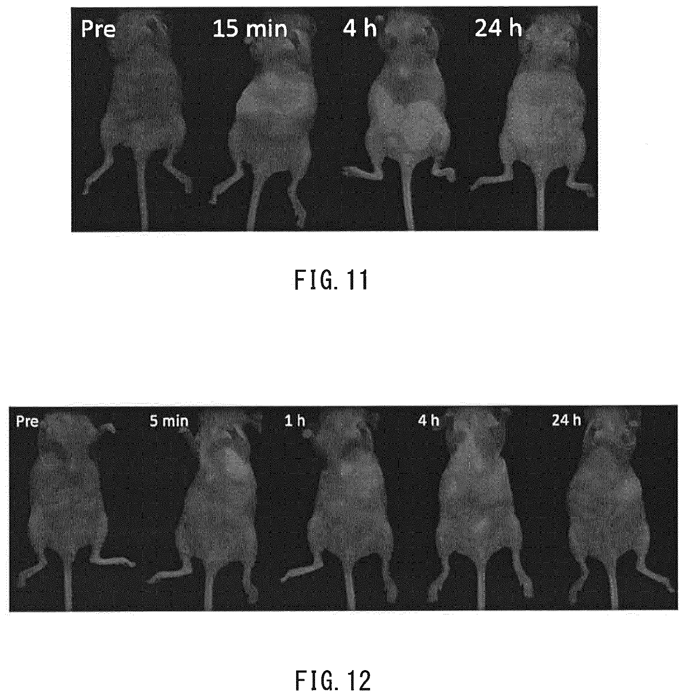

The fluorescence-labeled cells in the mouse body were observed using an in vivo fluorescence imaging apparatus "KURAVIC KV-700" manufactured by Kurabo Industries Ltd. In the observation, the timing just before the injection was taken as 0 hour, and photographs were taken of intervals until 24 hours had elapsed. The observation was performed using an excitation wavelength of 747 nm and a fluorescence wavelength of 850 nm based on the wavelength characteristics of ICG. The results were shown in FIGS. 11 (RAW264.7 cells) and 12 (HeLa S3 cells).

As shown in FIG. 11, the movement of the RAW264.7 cells labeled with the fluorescent probe (ICG-PPS HNPs) up to 24 hours could be confirmed. That is, it could be confirmed how the RAW264.7 cells moved in the mouse body. In particular, selecting the fluorescence wavelength suitable for in vivo observation made it possible to clearly capture only the fluorescence from the ICG without capturing autofluorescence. It was found that, after 24 hours, the RAW264.7 cells were distributed all through the internal organs though the amounts in the spleen and the liver were slightly larger.

As shown in FIG. 12, the movement of the HeLa S3 cells labeled with the fluorescent probe (FA-PEG/ICG-PPS HNPs) up to 24 hours could be confirmed. That is, it could be confirmed how the HeLa S3 cells moved in the mouse body. In particular, selecting the fluorescence wavelength suitable for in vivo observation made it possible to clearly capture only the fluorescence from the ICG without capturing autofluorescence. It was found that, after 24 hours, the cells accumulated particularly in the liver.
(4) Confirmation of accumulation of cells labeled with fluorescent probe in mouse body

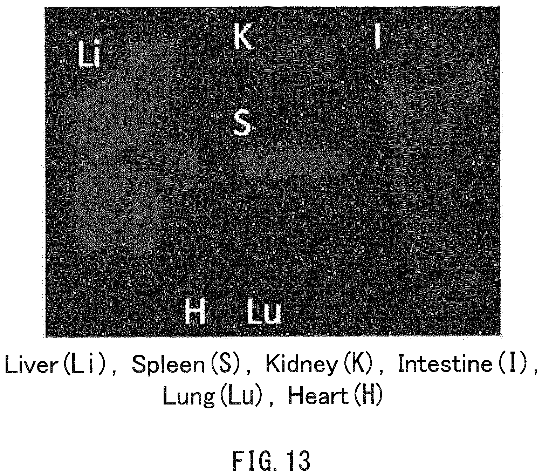

After 24 hours elapsed, the mouse was dissected, and the organs were removed. The accumulation state of the cells labeled with the fluorescent probe in the mouse body after 24 hours was observed using an in vivo fluorescence imaging apparatus "KURAVIC KV-700". The results are shown in FIGS. 13 (RAW264.7 cells), 14 (RAW264.7 cells), 15 (HeLa S3 cells), and 16 (HeLa S3 cells).

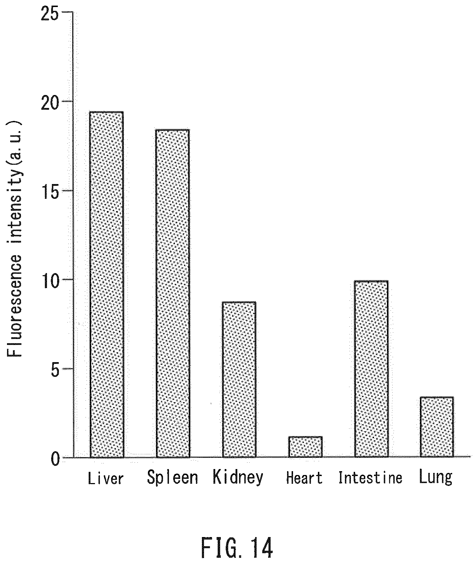

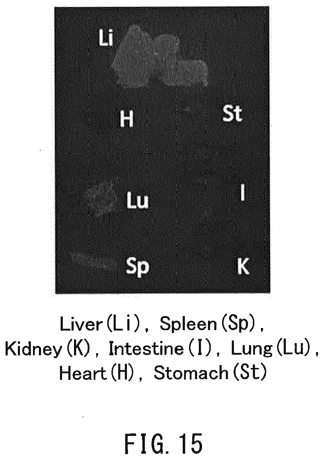

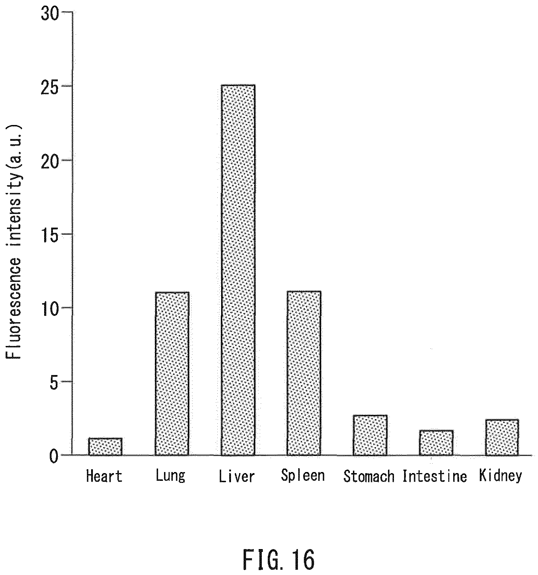

It was found from FIGS. 13 and 14 that, after 24 hours, the RAW264.7 cells labeled with the fluorescent probe (ICG-PPS HNPs) were distributed all through the internal organs though a slightly larger amount of the RAW264.7 cells accumulated in the spleen and the liver. It was found from FIGS. 15 and 16 that, after 24 hours, a particularly large amount of the HeLa S3 cells labeled with the fluorescent probe (FA-PEG/ICG-PPS HNPs) accumulated in the liver.

As is clear from the description above, using, as a fluorescent probe, a fluorescent probe such as the ICG-PPS HNPs or the FA-PEG/ICG-PPS HNPs, which includes two fluorescent dyes bound to a carrier molecule whose excitation wavelengths are different, and in which fluorescence resonance energy transfer does not occur between the two fluorescent dyes, namely fluorescent dyes a and b, makes it possible to observe cells labeled with the fluorescent probe using an in vitro fluorescence imaging apparatus such as a fluorescence microscope or a flow cytometer and to confirm the localization of the fluorescence-labeled cells over time in a living organism into which the cells labeled with the fluorescent probe were transplanted.

## Claims

1. A fluorescent probe comprising:
a carrier molecule;
a fluorescent dye a bound to the carrier molecule; and
a fluorescent dye b bound to the carrier molecule,
wherein excitation wavelengths of the fluorescent dyes a and b are different, and
fluorescence resonance energy transfer does not occur between the fluorescent dyes a and b.

2. The fluorescent probe according to claim 1, wherein the fluorescent probe is a cell labeling fluorescent probe, the fluorescent dye a is a fluorescent dye for in vitro fluorescence imaging, and the fluorescent dye b is a fluorescent dye for in vivo fluorescence imaging.

3. The fluorescent probe according to claim 1 or 2, which is specifically bound to a surface of a cell so that the cell is labeled.

4. The fluorescent probe according to claim 1 or 2, which is taken up by a cell so that the cell is labeled.

5. The fluorescent probe according to any one of claims 1 to 4, wherein an excitation wavelength of the fluorescent dye a is in a range of 350 to 650 nm.

6. The fluorescent probe according to any one of claims 1 to 5, wherein an excitation wavelength of the fluorescent dye b is in a range of 600 to 850 nm.

7. The fluorescent probe according to any one of claims 1 to 6, wherein the fluorescent dye a is porphyrin.

8. The fluorescent probe according to any one of claims 1 to 7, wherein the fluorescent dye b is indocyanine green.

9. The fluorescent probe according to any one of claims 1 to 8, wherein the carrier molecule is polysiloxane.

10. The fluorescent probe according to any one of claims 2 to 9, which is used in screening of fluorescence-labeled cells using a flow cytometer.

11. A method for detecting fluorescence used to detect target cells, comprising:
a step of labeling the target cells with a fluorescent probe; and
a step of irradiating the target cells labeled with the fluorescent probe with excitation light and observing fluorescence from the fluorescent probe,
wherein the fluorescent probe comprises a carrier molecule, a fluorescent dye a bound to the carrier molecule, and a fluorescent dye b bound to the carrier molecule,
excitation wavelengths of the fluorescent dyes a and b are different, and
fluorescence resonance energy transfer does not occur between the fluorescent dyes a and b.

12. The method for detecting fluorescence according to claim 11, wherein the fluorescent dye a is a fluorescent dye for in vitro fluorescence imaging, and the fluorescent dye b is a fluorescent dye for in vivo fluorescence imaging.

13. The method for detecting fluorescence according to claim 11 or 12,
wherein the target cells labeled with the fluorescent probe are irradiated with excitation light that excites the fluorescent dye a, and
fluorescence from the fluorescent probe is observed through in vitro fluorescence imaging.

14. The method for detecting fluorescence according to claim 11 or 12,
wherein the target cells labeled with the fluorescent probe are transplanted into a living organism (excluding a human),
the living organism is irradiated with excitation light that excites the fluorescent dye b, and
fluorescence from the fluorescent probe is observed through in vivo fluorescence imaging.

15. The method for detecting fluorescence according to any one of claims 11 to 14, wherein the fluorescent probe is specifically bound to a surface of a cell so that the cell is labeled.

16. The method for detecting fluorescence according to any one of claims 11 to 14, wherein the fluorescent probe is taken up by a cell so that the cell is labeled.

17. The method for detecting fluorescence according to any one of claims 11 to 16, wherein an excitation wavelength of the fluorescent dye a is in a range of 350 to 650 nm.

18. The method for detecting fluorescence according to any one of claims 11 to 17, wherein an excitation wavelength of the fluorescent dye b is in a range of 600 to 850 nm.

19. The method for detecting fluorescence according to any one of claims 11 to 18, wherein the fluorescent dye a is porphyrin.

20. The method for detecting fluorescence according to any one of claims 11 to 19, wherein the fluorescent dye b is indocyanine green.

21. A method for using the fluorescent probe according to any one of claims 1 to 10, the method comprising:
a step of fluorescently labeling cells with the fluorescent probe;
a step of confirming and screening the fluorescence-labeled cells that are fluorescently labeled with the fluorescent probe using a flow cytometer or a fluorescence microscope;
a step of transplanting the screened fluorescence-labeled cells into a living organism (excluding a human); and
a step of observing the fluorescence-labeled cells in the living organism using an in vivo fluorescence imaging apparatus,
wherein the screening of the fluorescence-labeled cells using a flow cytometer or a fluorescence microscope is performed by detecting fluorescence originating from the fluorescent dye a, and
the observation of the fluorescence-labeled cells in the living organism using an in vivo fluorescence imaging apparatus is performed by detecting fluorescence originating from the fluorescent dye b.
